(19) Europäisches Patentamt
European Patent Office
Office européen des brevets

(11) Veröffentlichungsnummer : **0 209 819**
**B1**

(12) # EUROPÄISCHE PATENTSCHRIFT

(45) Veröffentlichungstag der Patentschrift : **07.09.88**

(51) Int. Cl.⁴ : **C 07 C 121/34**, A 01 N 37/34

(21) Anmeldenummer : **86109582.6**

(22) Anmeldetag : **12.07.86**

(54) **3-(3-Iodpropargyloxy)-propionitril, ein Verfahren zu dessen Herstellung und dessen Verwendung.**

(30) Priorität : **26.07.85 DE 3526789**

(43) Veröffentlichungstag der Anmeldung : **28.01.87 Patentblatt 87/05**

(45) Bekanntmachung des Hinweises auf die Patenterteilung : **07.09.88 Patentblatt 88/36**

(84) Benannte Vertragsstaaten : **BE CH DE FR GB IT LI NL SE**

(56) Entgegenhaltungen :
**WO-A-84 /001 59**
**DE-A- 3 304 899**
**US-A- 2 809 988**
**CHEMICAL ABSTRACTS, Band 68, Nr. 11, 11. März 1968, Seite 4729, Zusammenfassung Nr. 49008b, Columbus, Ohio, US; M.F. SHOSTAKOVSKII et al.: "Cyanoethylation of acetylenic alcohols", & PROBL. POLUCH. POLUPROD. PROM. ORG. SIN., AKAD. NAUK SSSR, OTD. OBSHCH. TEKH. KHIM. 1967, 33-5**

(73) Patentinhaber : **BAYER AG**
**Konzernverwaltung RP Patentabteilung**
**D-5090 Leverkusen 1 Bayerwerk (DE)**

(72) Erfinder : **Schade, Gerold, Dr.**
**Hahnenweg 8**
**D-5000 Koeln 80 (DE)**
Erfinder : **Paulus, Wilfried, Dr.**
**Deswatinesstrasse 90**
**D-4150 Krefeld (DE)**
Erfinder : **Schmitt, Hans-Georg, Dr.**
**Am Oberend 13**
**D-4150 Krefeld 1 (DE)**

**EP 0 209 819 B1**

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft das neue 3-(3-Iodpropargyloxy)-propionitril, ein Verfahren zu dessen Herstellung sowie dessen Verwendung in mikrobiziden Mitteln.

Aus der DE-OS 3 224 503 und DE-OS 3 304 899 sind Mono-, Di- und Triethylenglykol-alkyl/aryl-(3-iod-2-propinyl)-ether bzw. substituierte 1-(3-Iod-2-propinyloxy-)-2- bzw. 3-propanole bekannt, die als antimikrobielle Mittel Verwendung finden.

Die in den genannten deutschen Offenlegungsschriften beschriebenen Verbindungen besitzen im allgemeinen zwar eine hohe mikrobizide Wirksamkeit, ihre Produktion und Verwendung in Konservierungsmitteln ist jedoch durch ihre physikalischen Eigenschaften stark beeinträchtigt. Die Verbindungen sind nämlich im allgemeinen nicht kristallin, sondern stellen Öle dar. Dadurch ist die Reinigung der Verbindungen erschwert. Eine Reinigung ist jedoch durchweg erforderlich, um produktionsbedingte Verfärbungen und Verunreinigungen zu entfernen, denn verfärbte Wirkstoffe können nicht zur Konservierung farbloser Produkte eingesetzt werden. Außerdem ist es aus toxikologischer Sicht wünschenswert, wenn die eingesetzten Verbindungen frei von Verunreinigungen sind.

Die bei Flüssigkeiten sonst übliche Reinigung durch Destillation ist bei den Iodpropargylverbindungen wie sie in den genannten deutschen Offenlegungsschriften beschrieben werden, im technischen Maßstab nicht durchführbar, da diese Verbindungen thermolabil sind und sich hierbei sogar explosionsartig zersetzen können (vgl. H. G. Viehe, Chemistry of Acetylenes, New York 1969, S. 691).

Überraschenderweise wurde nun gefunden, daß das neue Iodpropargyloxypropionitril der Formel I

$$IC{\equiv}C{-}CH_2{-}O{-}CH_2{-}CH_2{-}CN \qquad (I)$$

nicht nur eine hohe mikrobizide Wirksamkeit besitzt, sondern auch physikalische Eigenschaften hat, die die oben erwähnten Probleme nicht entstehen lassen. Das neue Iodpropargyloxypropionitril stellt einen kristallinen Feststoff von hohem Schmelzpunkt (66-68 °C) dar. Ein Aufschmelzen während der Produktion tritt nicht ein. Daneben besitzt die neue Verbindung eine so hohe Kristallisationstendenz, daß sie bei der Herstellung sofort kristallin und in hoher Reinheit anfällt. Falls es erforderlich ist, kann die neue Verbindung durch einfaches Umkristallisieren praktisch völlig rein hergestellt werden.

Die Herstellung des neuen Iodpropargyloxypropionitrils der Formel I erfolgt durch Iodierung von Propargyloxypropionitril der Formel II

$$HC{\equiv}C{-}CH_2{-}O{-}CH_2{-}CH_2{-}CN \qquad (II)$$

mit Iodierungsmitteln in Gegenwart von Lösungs- und/oder Verdünnungsmitteln und in Gegenwart von Basen bei Temperaturen von —10 °C bis + 30 °C.

Als Iodierungsmittel können in das erfindungsgemäße Verfahren Iod und/oder Iodidionen liefernde Verbindungen, wie Natriumiodid und Ammoniumiodid, in Gegenwart von Oxidationsmitteln, wie Natriumhypochlorit, Calciumhypochlorit und Wasserstoffperoxid, eingesetzt werden.

Als Basen eignen sich sowohl anorganische als auch organische Basen, wie Natriumhydroxid, Calciumhydroxid, Natriummethylat, Kalium-tert.-butylat und Natriumisobutylat, bevorzugt Natriumhydroxid und Natriummethylat.

Geeignete Lösungsmittel für das erfindungsgemäße Verfahren sind beispielsweise Wasser oder Alkohole, wie Methanol und/oder Ethanol, oder Gemische derselben. Bevorzugt wird die Iodierung bei Temperaturen von — 5 °C bis + 20 °C durchgeführt.

Erfindungsgemäß setzt man 1 mol Propargyloxypropionitril der Formel II mit etwa 1,0 bis 1,5 mol Iodierungsmittel, bevorzugt 1,0 bis 1,2 mol Iodierungsmittel ein.

Die jeweils günstigsten Mengen an Basen und an Lösungs- und/oder Verdünnungsmitteln lassen sich leicht durch Vorversuche ermitteln. Üblicherweise setzt man etwa 1 bis 3, bevorzugt 1,2 bis 2 mol Base pro mol Propargyloxypropionitril der allgemeinen Formel II und die gleiche bis fünffache, bevorzugt die doppelte bis dreifache, Gewichtsmenge an Lösungs- und/oder Verdünnungsmittel ein.

Das Propargyloxypropionitril der Formel II ist eine bekannte Verbindung (s. Chem. Abstr. 68, 49008). Diese Verbindung wird hergestellt durch Addition von Propargylalkohol an Acrylnitril in Gegenwart basischer Katalysatoren.

Das neue 3-(3-Iodpropargyloxy)-propionitril kann auf Grund seiner hohen mikrobiziden Wirksamkeit und seines breiten Wirkungsspektrums mit Vorteil zur Bekämpfung von Mikroorganismen, insbesondere zum Schutz technischer Materialien, verwendet werden. Es ist, wie die Vergleichsbeispiele zeigen, wesentlich effektiver als beispielsweise der aus der DE-OS 3 304 899 bekannte Iodpropargylether 1-(3-Iod-2-propinyloxy)-propan-2,3-diol.

Technische Materialien sind erfindungsgemäß nicht lebende Materialien, die für die Verwendung in der Technik zubereitet worden sind. Beispielsweise können technische Materialien, die durch den erfindungsgemäßen Wirkstoff vor mikrobieller Veränderung oder Zerstörung geschützt werden sollen, Klebstoffe, Leime, Papier und Karton, Textilien, Leder, Holz, Anstrichmittel und Kunststoffartikel, Kühlschmierstoffe und andere Materialien sein, die von Mikroorganismen befallen oder zersetzt werden

können. Im Rahmen der zu schützenden Materialien seien auch Teile von Produktionsanlagen, beispielsweise Kühlwasserkreisläufe, genannt, die durch Vermehrung von Mikroorganismen beeinträchtigt werden können. Im Rahmen der vorliegenden Erfindung seien als technische Materialien vorzugsweise Klebstoffe, Leime, Papiere und Kartone, Leder, Holz, Anstrichmittel, Kühlschmiermittel und Kühlkreisläufe genannt, besonders bevorzugt Holz.

Als Mikroorganismen, die einen Abbau oder eine Veränderung der technischen Materialien bewirken können, seien beispielsweise Bakterien, Pilze, Hefen, Algen und Schleimorganismen genannt. Vorzugsweise wirkt der erfindungsgemäße Wirkstoff gegen Pilze, insbesondere Schimmelpilze, holzverfärbende und holzzerstörende Pilze (Basidiomyceten), sowie gegen Schleimorganismen und Algen.

Es seien beispielsweise Mikroorganismen der folgenden Gattungen genannt :

Alternaria, wie Alternaria tenuis,
Aspergillus, wie Aspergillus niger,
Chaetomium, wie Chaetomium globosum,
Coniophora, wie Coniophora puteana,
Lentinus, wie Lentinus tigrinus,
Penicillium, wie Penicillium glaucum,
Polyporus, wie Polyporus versicolor,
Aureobasidium, wie Aureobasidium pullulans,
Sclerophoma, wie Sclerophoma pityophila,
Trichoderma, wie Trichoderma viride,
Escherichia, wie Escherichia coli,
Pseudomonas, wie Pseudomonas aeruginosa,
Staphylococcus, wie Staphylococcus aureus.

Je nach Anwendungsgebiet kann der erfindungsgemäße Wirkstoff in die üblichen Formulierungen überführt werden, wie Lösungen, Emulsionen, Suspensionen, Pulver, Pasten und Granulate.

Diese können in an sich bekannter Weise hergestellt werden, z. B. durch Vermischen des Wirkstoffs mit einem Streckmittel, das aus flüssigem Lösungsmittel und/oder festen Trägerstoffen besteht, gegebenenfalls unter Verwendung von oberflächenaktiven Mitteln, wie Emulgatoren und/oder Dispergiermitteln, wobei gegebenenfalls im Falle der Benutzung von Wasser als Streckmittel organische Lösungsmittel wie Alkohole als Hilfsmittel verwendet werden können.

Flüssige Lösungsmittel für den Wirkstoff können beispielsweise Wasser, Alkohole, wie niedere aliphatische Alkohole, vorzugsweise Ethanol oder Isopropanol, oder Benzylalkohol, Ketone, wie Aceton oder Methylethylketon, flüssige Kohlenwasserstoffe, wie Benzinfraktionen, halogenierte Kohlenwasserstoffe, wie 1,2-Dichlorethan, sein.

Mikrobizide Mittel enthalten den Wirkstoff im allgemeinen in einer Menge von 1 bis 95 %, bevorzugt von 10 bis 75 %. Die Anwendungskonzentration des erfindungsgemäßen Wirkstoffs richten sich nach der Art und dem Vorkommen der zu bekämpfenden Mikroorganismen, sowie nach der Zusammensetzung des zu schützenden Materials. Die optimale Einsatzmenge kann durch Testreihen ermittelt werden. Im allgemeinen liegen die Anwendungskonzentrationen im Bereich von 0,001 bis 5 Gew.-%, vorzugsweise von 0,05 bis 1,0 Gew.-%, bezogen auf das zu schützende Material.

Der erfindungsgemäße Wirkstoff kann auch in Mischung mit anderen bekannten Wirkstoffen vorliegen. Beispielsweise seien die folgenden Wirkstoffe genannt : Benzylalkoholmono-(poly)hemiformal und andere Formaldehyd abspaltende Verbindungen, Benzimidazolyl-methylcarbamate, Tetramethylthiuramdisulfid, Zinksalze von Dialkyldithiocarbamaten, 2,4,5,6-Tetrachlorisophthalonitril, Thiazolylbenzimidazol, Mercaptobenzthiazol, 2-Rhodanidomethylthio-benzthiazol, Organo-Zinnverbindungen, Methylenbisthiocyanat, Phenolderivate, wie 2-Phenylphenol, (2,2'-Dihydroxy-5,5'-dichlor)-diphenylmethan und 3-Methyl-4-chlor-phenol.

## Herstellungsbeispiel

### Beispiel 1

86,3 g (0,8 mol) Propargyloxypropionitril, 71 g 50 %ige NaOH und 500 ml Methanol werden bei 0 bis 5 °C portionsweise mit 230 g (0,9 mol) Iod versetzt. Man rührt 1 h nach, entfärbt mit $NaHSO_3$-Lösung, gießt in 1,5 l Eiswasser und saugt ab. Ausbeute 154 g (83 %), schwach gelbliche Kristalle, Schmp. 65-68 °C, Reinheit 98,5 % (GC).

Nach Umkristallisieren aus Cyclohexan farblose Kristalle, Schmp. 66-68 °C, Reinheit 100 % (GC).

## Anwendungsbeispiele

### Beispiel 2

Zum Nachweis der Wirksamkeit gegen Pilze werden die minimalen Hemm-Konzentrationen (MHK)

von erfindungsgemäßen Wirkstoffen bestimmt:

Ein Agar, der aus Bierwürze und Pepton hergestellt wird, wird mit erfindungsgemäßen Wirkstoffen in Konzentrationen von 0,1 mg/l bis 500 mg/l versetzt. Nach Erstarren des Agars erfolgt Kontamination mit Reinkulturen der in der Tabelle aufgeführten Testorganismen. Nach 2-wöchiger Lagerung bei 28 °C und 60 bis 70 % rel. Luftfeuchtigkeit wird die MHK bestimmt. MHK ist die niedrigste Konzentration an Wirkstoff, bei der keinerlei Bewuchs durch die verwendete Mikrobenart erfolgt, sie ist in der nachstehenden Tabelle angegeben.

Tabelle 1 : MHK's in mg/l bei der Einwirkung erfindungsgemäßer Substanzen auf Pilze

| | Substanz | |
|---|---|---|
| Testorganismen | erfindungsgem. Substanz | Vergleichssubstanz* |
| Alternaria tenuis | 5 | 50 |
| Aspergillus niger | 5 | 50 |
| Aureobasidium pullulans | 5 | 50 |
| Chaetomium globosum | 20 | 100 |
| Cladeosporium cladesporioides | 5 | |
| Lentinus tigrinus | 15 | 50 |
| Penicillium glaucum | 5 | 50 |
| Sclerophoma pityophila | 5 | 50 |
| Trichoderma viride | 20 | 500 |

(*) 1-(3-Iod-2-propinyloxy)-propan-2,3-diol/Verbindung des Beispiels 73 der DE-OS 3 304 899.

Beispiel 3 (Wirkung gegen Schleimorganismen)

Die erfindungsgemäße Substanz wird in Konzentrationen von jeweils 0,1 bis 100 mg/l in Allens Nährlösung (Arch. Mikrobiol. 17, 34 bis 53 (1952)), die in 4 l sterilem Wasser, 0,2 g Ammoniumchlorid, 4,0 g Natriumnitrat, 1,0 g Dikaliumhydrogenphosphat, 0,2 g Calciumchlorid, 2,05 g Magnesiumsulfat, 0,02 g Eisenchlorid und 1 % Caprolactam enthält, in wenig Aceton gelöst, zur Anwendung gebracht. Kurz vorher wird die Nährlösung mit Schleimorganismen (ca. $10^6$ Keime/ml), die aus bei der Polyamid-Herstellung verwendeten Spinnwasser-Kreisläufen isoliert wurden, infiziert. Nährlösungen, die die minimale Hemmkonzentration (MHK) oder größere Wirkstoffkonzentrationen aufweisen, sind nach 3-wöchiger Kultur bei Raumtemperatur noch völlig klar, d. h. die in wirkstofffreien Nährlösungen nach 3 bis 4 Tagen bemerkbare starke Vermehrung der Mikroben und Schleimbildung unterbleibt.

Tabelle II

MHK-Werte in mg/l bei der Einwirkung der unten angegebenen Substanzen auf Schleimorganismen

| Wirkstoff | MHK in mg/l |
|---|---|
| erfindungsgemäß | 15 |
| Vergleichssubstanz = 1-(3-Iod-2-propinyloxy)-propan-2,3-diol | 35 |

**Patentansprüche**

1. 3-(3-Iodpropargyloxy)-propionitril der Formel

$$IC{\equiv}C{-}CH_2{-}O{-}CH_2{-}CH_2{-}CN\ .$$

2. Verfahren zur Herstellung des 3-(3-Iodpropargyloxy)-propionitrils, dadurch gekennzeichnet, daß man Propargyloxypropionitril der Formel

$$HC{\equiv}C—CH_2—O—CH_2—CH_2—CN$$

mit Iodierungsmitteln in Gegenwart von Lösungs- und/oder Verdünnungsmitteln und in Gegenwart von Basen bei Temperaturen von —10 bis + 30 °C umsetzt.

3. Mikrobizides Mittel, enthaltend das 3-(3-Iodpropargyloxy)-propionitril der Formel

$$IC{\equiv}C—CH_2—O—CH_2—CH_2—CN\ .$$

4. Mikrobizides Mittel nach Anspruch 3, enthaltend 1 bis 95 Gew.-% des Iodpropargyloxypropionitrils.

5. Verwendung des mikrobiziden Mittels nach den Ansprüchen 3 und 4 zum Schutz technischer Materialien.

6. Verwendung des mikrobiziden Mittels nach Anspruch 5 zum Schutz von Holz gegen holzverfärbende und holzzerstörende Pilze.

**Claims**

1. 3-(3-Iodopropargyloxy)-propionitrile of the formula

$$IC{\equiv}C—CH_2—O—CH_2—CH_2—CN\ .$$

2. Process for the preparation of 3-(3-iodopropargyloxy)-propionitrile, characterized in that propargyloxy-propionitrile of the formula

$$HC{\equiv}C—CH_2—O—CH_2—CH_2—CN$$

is reacted with iodinating agents in the presence of solvents and/or diluents and in the presence of bases at temperatures from —10 to + 30 °C.

3. Microbicidal agent containing 3-(3-iodopropargyloxy)-propionitrile of the formula

$$IC{\equiv}C—CH_2—O—CH_2—CH_2—CN\ .$$

4. Microbicidal agent according to Claim 3, containing 1 to 95 % by weight of iodopropargyloxypropionitrile.

5. Use of the microbicidal agent according to Claims 3 and 4 for the preservation of industrial materials.

6. Use of the microbicidal agent according to Claim 5 for the preservation of wood from fungi which discolour and destroy wood.

**Revendications**

1. Le 3-(3-iodopropargyloxy)-propionitrile de formule

$$IC{\equiv}C—CH_2—O—CH_2—CH_2—CN\ .$$

2. Procédé de préparation du 3-(3-iodopropargyloxy)-propionitrile, caractérisé en ce qu'on fait réagir du propargyloxypropionitrile de formule

$$HC{\equiv}C—CH_2—O—CH_2—CH_2—CN$$

avec des agents d'iodation en présence de solvants et/ou de diluants et en présence de bases, à des températures de —10 à + 30 °C.

3. Composition microbicide, contenant le 3-(3-iodopropargyloxy)-propionitrile de formule

$$IC{\equiv}C—CH_2—O—CH_2—CH_2—CN\ .$$

4. Composition microbicide suivant la revendication 3, contenant 1 à 95 % en poids d'iodopropargyloxypropionitrile.

5. Utilisation de la composition microbicide suivant les revendications 3 et 4, pour la protection de matériaux industriels.

6. Utilisation de la composition microbicide suivant la revendication 5, pour protéger le bois de champignons qui en modifient la couleur et qui le détruisent.